# EUROPEAN PATENT SPECIFICATION

(11) **EP 2 833 819 B1**
(45) Date of publication and mention of the grant of the patent: **14.10.2015**
(21) Application number: 13742516.1
(22) Date of filing: 10.06.2013
(51) Int. Cl.: A61B 18/20

(54) **LIOB BASED SKIN TREATMENT SYSTEM**
AUF LIOB BASIERENDES HAUTBEHANDLUNGSSYSTEM
SYSTÈME DE TRAITEMENT DE LA PEAU À BASE DE LIOB

(30) Priority: 14.06.2012 US 201261659477 P
(43) Date of publication of application: 11.02.2015
(73) Proprietor: Koninklijke Philips N.V., 5656 AE Eindhoven (NL)
(72) Inventor: MOESKOPS, Bastiaan, Wilhelmus, Maria, 5656 AE Eindhoven (NL); THUMMA, Kiran, Kumar, 5656 AE Eindhoven (NL); PULLY, Vishnu, Vardhan, 5656 AE Eindhoven (NL); VERHAGEN, Rieko, 5656 AE Eindhoven (NL)
(74) Representative: Coops, Peter
(86) International application number: PCT/IB2013/054740
(87) International publication number: WO 2013/186686

(56) References cited:
- EP-A1- 2 030 586
- WO-A1-98/47435
- WO-A2-2011/094742
- DE-A1- 2 832 847
- US-A1- 2004 082 941

## Description

### FIELD OF THE INVENTION

This invention relates to a skin treatment system comprising a light based skin treatment device comprising a treatment light source for providing a treatment light beam for treating hair or skin tissue, and a transparent exit window for allowing the treatment light beam to leave the device.

The invention further relates to a docking station for use in such a system.

### BACKGROUND OF THE INVENTION

Such light based skin treatment devices are, e.g., used for laser induced optical breakdown (LIOB) based wrinkle treatment or shaving. In light based wrinkle treatment, the device creates a focal spot in a dermis layer of the skin to be treated. The power of the laser is selected such that LIOB affects the skin in order to stimulate re-growth of skin tissue and, therewith, to reduce wrinkles. In a light based shaver, the incident light beam is focused inside the hair and the LIOB causes the hair to be cut.

For example, the international patent application published as WO 2005/011510 describes such a device for shortening hairs comprising a laser source for generating a laser beam during a predetermined pulse time, an optical system for focusing the laser beam into a focus spot and a laser beam manipulator for positioning the focal spot in the target position. A dimension of the focal spot and a power of the generated laser beam are such that in the focal spot the laser beam has a power density which is above a characteristic threshold value for hair tissue above which, for the predetermined pulse time, a laser induced optical breakdown (LIOB) phenomenon occurs in the hair tissue.

In general, laser induced optical breakdown (LIOB) occurs in media, which are transparent or semi-transparent for the wavelength of a pulsed laser beam, when the power density of the laser beam in the focal spot exceeds a threshold value which is characteristic for the particular medium. Below the threshold value, the particular medium has relatively low linear absorption properties for the particular wavelength of the laser beam.

Above the threshold value, the medium has strongly non-linear absorption properties for the particular wavelength of the laser beam, which are the result of ionization of the medium and the formation of plasma. This LIOB phenomenon results in a number of mechanical effects, such as cavitation and the generation of shock waves, which damage the medium in positions surrounding the position of the LIOB phenomenon.

From experiments it appeared that the LIOB phenomenon can be used to break and shorten hairs growing from skin. Hair tissue is transparent or semi-transparent for wavelengths between approximately 500 nm and 2000 nm. For each value of the wavelength within this range, LIOB phenomena occur in the hair tissue at the location of the focal spot when the power density of the laser beam in the focal spot exceeds a threshold value which is characteristic for the hair tissue. Said threshold value is rather close to the threshold value which is characteristic for aqueous media and tissue and is dependent on the pulse time of the laser beam. In particular, the threshold value of the required power density (W/cm²) decreases when the pulse time increases. It appeared that, in order to achieve mechanical effects as a result of the LIOB phenomenon which are sufficiently effective so as to cause significant damage, i.e. at least initial breakage of a hair, a pulse time in the order of, for example, 10 ns suffices. For this value of the pulse time, the threshold value of the power density of the laser beam in the focal spot is in the order of 2*10¹⁰ W/cm². For the described pulse time and with a sufficiently small focal spot size obtained, for example, by means of a lens having a sufficiently large numerical aperture, this threshold value can be achieved with a total pulse energy of only a few tenths of a mJ.

Whilst it is possible using the device of WO 2005/011510 to generate a laser induced optical breakdown (LIOB) from an incident beam leaving the device through a small glass "blade" and with sufficient energy to cut human hairs, the products of the LIOB (shock wave, plasma, high irradiance) can cause destructive damage of the blade. A damaged blade has a detrimental effect on the ability of the device to provide a tight focus at the desired position, which may reduce the efficacy of the shaving process and/or may increase the occurrence of adverse side effects, such as skin irritation. Similar problems with LIOB caused damages to the exit window may occur in light based wrinkle devices for wrinkle treatment.

DE 28 32 847 teaches a system according to the preamble of claim 1.

### OBJECT OF THE INVENTION

It is an object of the invention to provide a skin treatment system wherein the effect of LIOB caused damages is reduced.

### SUMMARY OF THE INVENTION

According to a first aspect of the invention, this object is achieved by providing a skin treatment system comprising a light based skin treatment device, a detection light source, a detection unit and a control circuit. The light based skin treatment device comprises a treatment light source for providing a treatment light beam for treating hair or skin tissue, a transparent exit window for allowing the treatment light beam to leave the device, and an exit location control unit for controlling a position for the treatment light beam to pass through the exit window. The detection light source is provided for providing a detection light beam. The detection unit is provided for detecting the detection light beam after having passed at least partially through the exit window at least once. The control circuit is arranged to analyze a detection signal from the detection unit to find damaged areas at an outer surface of the exit window, and to control the exit location control unit in order to avoid the treatment light beam to pass through the damaged areas.

If the skin treatment device uses LIOB phenomena for treating hair or skin tissue, the damages may be caused by the LIOB phenomena. In the following the invention will be described with reference to LIOB based treatment and LIOB caused damages. It is, however, to be noted that light based skin treatment is also possible without using LIOB and that damages may also occur due to other causes. By preventing that the treatment light beam passes the damaged sections of the outer surface of the exit window, unpredictable scattering events at the outer surface of the exit window are averted. Unexpected scattering behavior may, e.g., lead to LIOB phenomena taking place at positions where they are not desired or may impede the creation of LIOB at positions where it is. Furthermore, LIOB taking place close to an already damaged area of the outer section may be even more destructive than when occurring close to unaffected areas.

One common light source may be used for providing both the treatment light beam and the detection light beam. The treatment light beam and the detection light beam preferably have similar or equal optical characteristics and the same incident beam may simultaneously be used for treatment as well as for detection. In the following, unless stated otherwise, we will assume that the system has one light source and provides only one incident light beam. It is, however, to be noted that the detection light beam may, e.g., have a different intensity, wavelength or pulse pattern than the treatment light beam.

The exit location control unit may be realized in one or more of the following ways. The exit window may be moved to a new position, such that the light beam will pass through a different part of the exit window. The treatment light source itself may be displaced relative to the exit window for a similar result. Alternatively, focusing elements, such as mirrors and/or lenses, may be rotated or displaced for changing the path of the treatment light beam to the skin in such a way that the light beam does not have to pass through damaged sections of the outer surface.

When the exit location control unit is arranged for scanning the treatment light beam over an area of the skin, it may prevent that the treatment light beam passes through damaged area of the exit window by activating and deactivating the treatment light source accordingly. For example, the treatment light source may be deactivated when the exit location control unit is arranged such that activation of the treatment light source would have caused the treatment light beam to pass through the damaged areas.

In an embodiment of the system according to the invention, the control circuit is arranged to analyze at least two detection signals from the detection unit, generated with the detection light beam passing through the exit window at the same or almost the same position at at least two respective moments in time, and to declare a certain area at the outer surface as damaged if the at least two detection signals represent a similar aberrant scattering profile of the detection light beam at the outer surface.

Aberrant herein means that the scattering profile is significantly different from the scattering profile that is to be expected from a clean and undamaged exit window surface. Damages and contaminations at the outer surface lead to changes in the scattering profile. Contaminations that may stick to the outer surface of the exit window, like hairs, skin, dirt or debris, will have a dynamic nature and will not remain constant over time. Damages to the outer surface do not change position relative to the exit window itself. Between the two measurements, e.g., movement of the skin treatment device over the skin surface or shockwaves created by LIOB may remove unwanted features from the outer surface. A localized damage, however, causes a constant feature that can be recognized when analyzing the two detection signals representing different moments in time.

The detection signal is generated by the portion of the detection light beam that is scattered from the exit window. Typically, intensity of this scattered light at one or more wavelengths would be measured. A 'clean' spot on the exit window results in a scattering signal that is close to the scattering signal in a previously determined 'virgin' state, e.g. measured at factory. Over time, two effects may occur, contamination (temporary), or damage (permanent). Damage can be distinguished from contamination by measuring at different time points. One option is to take a measurement before and after a cleaning cycle, or on separate days. In this way, it is likely that contaminations are fully removed or partially removed. Therefore, the intensity of the scattered light, although initially lower than the 'virgin' state, changes over time, indicating contamination of a temporary nature. If the intensity of the measured scattering is both significantly lower than the original 'virgin' state, and does not change (recover) over time, then it may be concluded that this section of the exit window is damaged. The corresponding section may then be excluded from further use. Alternatively, the system can bring the exit window back to a 'virgin' state by a cleaning step, in which all contaminants are removed. Then, the scattering signal is measured at a single point in time. If the signal is significantly different from the expected 'virgin' state signal, it can be concluded that the exit window is damaged. Combinations of the aforementioned methods may of course also be used (cleaning step + multiple measurements at different points in time).

Consecutive line scans may be made for scanning the whole or parts of the outer surface. During a line scan the position where the light beam passes through the exit window moves along a predetermined line. Two or more consecutive line scans may follow exactly the same trajectory or may, e.g., cover two parallel lines with only a small distance in between. Two positions at the outer surface of the exit window are considered to be 'almost at the same position' when they are separated by a distance of at most the typical size of a minor damage to the outer surface. The two respective moments in time should be separated long enough for temporary contaminants to disappear. The outer surface is preferably cleaned in between the two respective moments. Alternatively, the contaminants are removed due to dragging the outer surface along the skin or LIOB caused shockwaves.

The detection light source, the detection unit and part of the control circuit may either be comprised in the skin treatment device itself or in a separate docking station for receiving the skin treatment device. When the treatment light source is also used for detection purposes, the docking station does not need its own light source. When a separate detection light source is used, it may either be comprised in the treatment device or in the docking station. The docking station may further comprise means for electrically charging the skin treatment device and/or for cleaning the outer surface. The detection unit in the docking station should be coupled to the control unit and the exit location control unit of the treatment device for making it possible to avoid the light beam having to pass through the damaged sections. This coupling may be realized using wired or wireless communication means. If the skin treatment device comprises a hair detector, then this detector may also be used as the detection unit that is used for detecting the damaged sections.

According to further aspects of the invention, a docking station for use in the above described system is provided.

These and other aspects of the invention are apparent from and will be elucidated with reference to the embodiments described hereinafter.

### BRIEF DESCRIPTION OF THE DRAWINGS

In the drawings:
Figure 1 schematically shows a laser shaver according to the invention.
Figure 2 schematically shows a skin rejuvenation device according to the invention, and
Figure 3 schematically shows a combination of a skin rejuvenation device and a docking station.

### DETAILED DESCRIPTION OF THE INVENTION

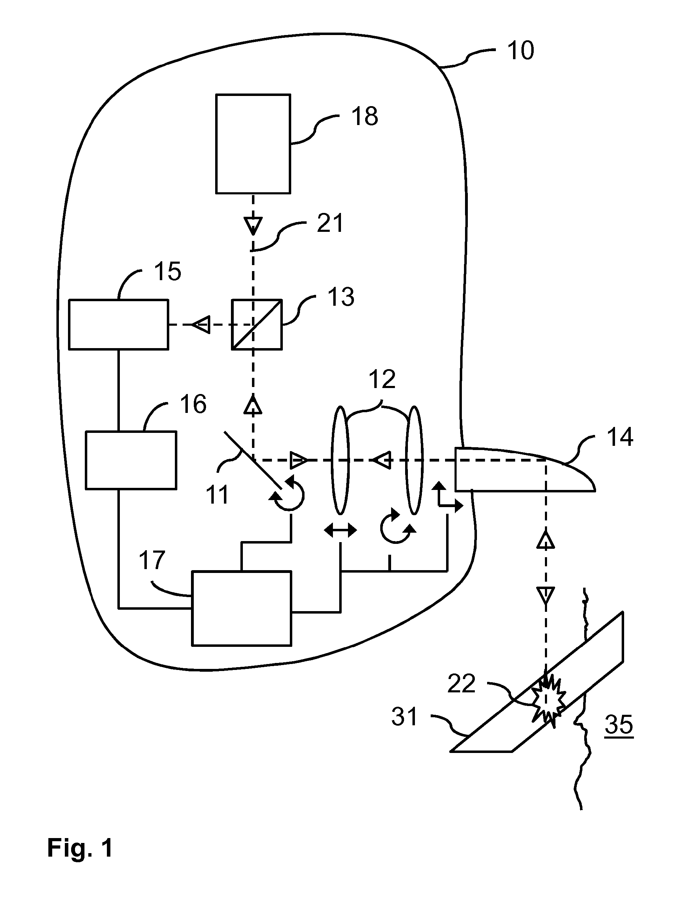

Figure 1 schematically shows a laser shaver 10 according to the invention. The laser shaver 10 is an example of a skin treatment device using laser induced optical breakdown (LIOB) for skin treatment. In this example, the skin treatment consists of optically cutting hairs 31 by focusing a laser beam 21 with sufficient power inside the hairs 31. The laser beam 21 is provided by a laser source 18, which may comprise a laser diode. For example, pulsed Nd:YAG lasers with emission at 1064 nm or Er:YAG lasers with emission at 1645 nm are used for LIOB cutting of the hairs 31.

The light beam 21 leaves the laser shaver 10 via the transparent exit window. In this laser shaver 10 the exit window is embodied as an optical blade 14 which also directs the light beam 21 in a direction, more or less parallel to the skin 35 (when the device is used). A processor (not shown) is provided and coupled to, e.g., the light source 18 and the focusing device 17 for controlling their operation.

Optical elements, such as lenses 12, mirrors 11 and the optical blade 14 are provided for focusing the pulsed laser beam 21 in the hair 31. The optical elements 11, 12, 14 may be adjustable by a focusing device 17 for adapting the exact position of the focal point 22 when needed. The focusing device 17 may adjust the exact trajectory of the light beam 21 and position of the focus by rotating and/or displacing the optical elements 11, 12. By adjusting the trajectory of the light beam 21, also the exact position where the light beam 21 leaves the exit window 14 changes. The focusing device 17 for controlling the optical elements 11, 12, 14 may thus also be considered to be an exit location control unit 17. In the example, one control unit operates the configuration of all the optical elements 11, 12, 14. Of course, separate control units may be provided for configuring, e.g., only the lens 12 positions or mirror 11 orientations. Alternatively, the functions of the focusing device 17 are integrated in a central processing unit (not shown) for controlling the overall operation of the device 10. It is to be noted that instead of (or in addition to) controlling the trajectory of the light beam 21, it is also an option to move the exit window 14 relative to the housing of the device 10.

Part of the light 21 reaching the skin 35 or hair 31 is reflected or scattered at the skin 35 or hair 31 tissue and reenters the device 10 via the exit window 14. The laser shaver 10 of figure 1 additionally comprises a detection unit 15 for detecting the light beam 21 after having been reflected, scattered or otherwise interacted at the skin 35 or hair 31 tissue. The detection unit 15 may comprise one or more photodiodes. The detection unit may be arranged to detect only light having a predefined polarization direction. It may also comprise multiple channels for separately measuring different polarization components. Such detection units 15 are usually provided in laser shavers 10 for performing hair detection. In this example, such a detection unit 15 is further used for the detection of damages in the outer surface of the exit window/optical blade 14. The reflected light is detected by the detector 15 and a detection signal representing the detected light is provided to a control circuit 16. The control circuit may be an integrated part of a central processing unit (not shown) for controlling the overall operation of the device 10. The control circuit 16 analyzes the detection signal to determine whether there are damaged areas at the outer surface of the exit window 14. When the control circuit 16 finds damaged areas at the outer surface of the exit window 14, the focusing device 17 controls the configuration of one or more of the optical elements 11, 12, 14 such that the light beam 21 can pass through the exit window 14 without hitting the damaged area or damaged areas. The control circuit may further be arranged to control the focusing device 17 to adjust a position or configuration of the light source 18 in order to adjust the trajectory of the light beam 21 and the location where the light beam 21 crosses the outer surface of the exit window 14.

During the detection process, the focusing device 17 may be used for scanning the whole or part of the outer surface with the light beam 21. The positions where surface damage is detected may be stored in a database. During use of the laser shaver 10, the content of such a database may be used for, later on, avoiding that the light beam 21 has to pass through such damaged areas. For reliably discriminating between surface damage and removable dirt, a certain area of the outer surface may only be declared defect if a damage is detected at the same position at least twice with a sufficiently long time span in between the two moments of detection. In principle, only static features detected at the outer surface are to be registered as damages. All detected features of a dynamic nature are probably caused by contamination.

The detection of damaged areas at the outer surface of the exit window 14 may be performed during normal use of the laser shaver 10 or as a separate process before or after the normal use. When the detection light beam for finding damaged areas is not the same beam as the treatment beam (temporarily separated and/or arising from a separate light source), different wavelengths, intensities or pulse patterns may be used. In order to ensure that skin parts, hair, water or other dirt or liquids is not interpreted as a damage to the outer surface, the exit window may be cleaned before performing the detection process. A reflective sheet may be applied to the outer surface of the exit window 14 to make the light beam 21 reflect on an equal surface.

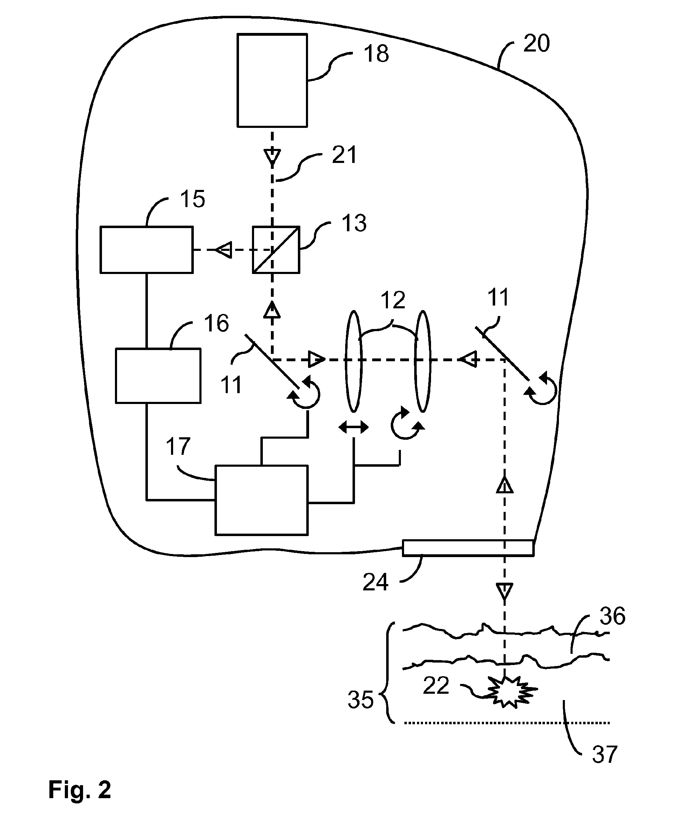

Figure 2 schematically shows a skin rejuvenation device 20 according to the invention. The skin rejuvenation device 20 is similar to the laser shaver 10 of figure 1 in many aspects. Instead of the optical blade 14, now an exit window 24 is provided for directing the light beam 21 directly towards the skin 35. Human or animal skin 35 comprises multiple layers 35, 36 of skin tissue. The outer surface of the skin 35 is called the epidermis 36. Underneath the epidermis 36, a collagen rich dermis layer 37 is located. The skin rejuvenation device 20 is configured to create LIOB events 22 inside the collagen of the dermis 37 in order to create small skin lesions, thereby prompting the skin 35 to heal itself by creating new cells. This process leads to a reduction of wrinkles in the skin 35.

The detection unit 15 in this skin rejuvenation device 20 detects light reentering the device 20 in the same way as the detection unit 15 of the laser shaver 10 of figure 1. However, because the skin rejuvenation device 16 does not need to detect hairs, the detection unit 15 may not be arranged to detect hairs. Instead, the detection unit may additionally be used for detecting a collagen content of the skin 35 at the focus depth. Focus depth may then be adapted in order to be sure that the LIOB events 22 are created at an optimal depth inside the skin 35. The control circuit 16 and the focusing device 17 operate as already described above with reference to figure 1.

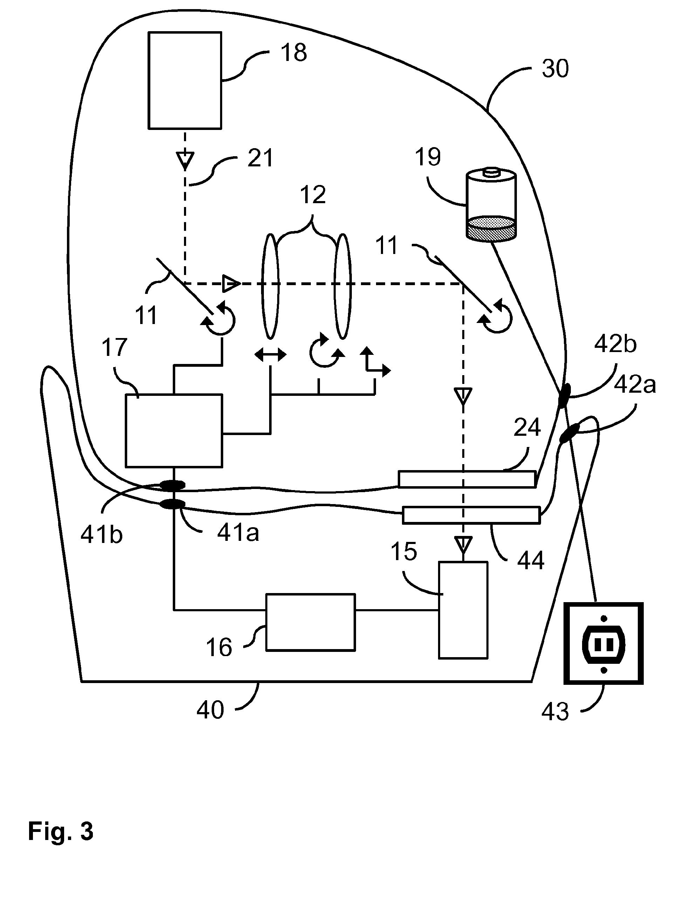

Figure 3 schematically shows a combination of a skin rejuvenation device 30 and a docking station 40. The skin rejuvenation device 30 of figure 3 is similar to the device 20 of figure 2. However, this skin rejuvenation device 30 does not comprise a light detection unit 15. The docking station 40 is provided for putting the skin rejuvenation device 30 into when it is not being used. When put into the docking station 40, a rechargeable battery unit 19 of the device 30 may be charged via a connection to a power grid 43. This connection is provided by two electrical contacts 42a, 42b provided at the docking station 40 and the skin rejuvenation device 30 respectively, which contacts 42a, 42b are arranged such that they couple the battery 19 to the grid 43 when the device 30 is placed in the docking station 40.

Instead of a detection unit in the skin treatment device itself (as in figures 1 and 2), now a detection unit 15 is comprised in the docking station 40. The detection unit 15 receives the light beam 21 after having passed through the exit window 24 of the skin rejuvenation device 30. The docking station may comprise an entrance window 44 for protecting the detection unit 15 against damage or contamination. Further optical elements may be provided in the docking station for focusing the light beam 21 into the detection unit 15. The detection unit 15 and the control circuit 16 are used for determining whether the outer surface of the exit window 24 is damaged. The results of the damage detection process are provided to the focusing device 17 in the skin rejuvenation device 30 via electrical contacts 41a, 41b or via wireless communication. Alternatively, the control circuit 16 may be comprised in the skin treatment device 30 and the detection signal is provided to the control circuit 16 via the electrical contacts 41a, 41b or wireless communication. It is also possible to perform some of the tasks of the control circuit, e.g. the determining of damaged areas, in the docking station and to perform other tasks, e.g. deciding how to avoid the damaged areas, in the treatment device.

If separate light sources are used for the treatment light beam and the detection light beam, the detection light source for providing the detection light beam may be provided in the docking station 40 instead of in the treatment device. In that event, a detection unit 15 in the docking station 40 may detect the detection light beam after reflection at the exit window 24 of the treatment device and/or a detection unit 15 in the treatment device may detect the detection light beam after having passed through the exit window 24.

The docking station may additionally comprise a cleaning unit (not shown) for cleaning the outer surface of the exit window 24 before performing the detection process. If the docking station 40 comprises an entrance window 44, also cleaning of the entrance window 44 may be important. The entrance window 44 may be replaced by a new or different one now and then in order to be sure that a detected damage is really part of the exit window 24 of the device 30 and not of the entrance window 44 of the docking station. Alternatively, the entrance window 44 changes position in between two attempts to detect damages on the exit window 24 of the device 30. Alternatively, sensing technology can be used to distinguish the optical signals coming from the two windows (e.g. distance sensing).

It is to be noted that also the laser shaver 10 of figure 1 and the skin rejuvenation device 20 of figure 2 may be provided in combination with a docking station.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A skin treatment system comprising:
- a light based skin treatment device (10, 20, 30) comprising
a treatment light source (18) for providing a treatment light beam (21) for treating hair or skin tissue,
a transparent exit window (14) for allowing the treatment light beam (21) to leave the device (10, 20, 30), and
an exit location control unit (17) for controlling a position for the treatment light beam (21) to pass through the exit window (14),
- a detection light source (18) for providing a detection light beam (21),
- a detection unit (15) for detecting the detection light beam (21) after having passed at least partially through the exit window (14) at least once,
**characterised by**
- a control circuit (16) arranged to
- analyze a detection signal from the detection unit (15) to find damaged areas at an outer surface of the exit window (14), and to
- control the exit location control unit (17) in order to avoid the treatment light beam (18) to pass through the damaged areas.

2. A skin treatment system as claimed in claim 1, wherein the treatment light source and the detection light source are the same light source (18).

3. A skin treatment system as claimed in claim 2, wherein the treatment light beam and the detection light beam are the same incident light beam (21).

4. A skin treatment system as claimed in claim 1, wherein the exit location control unit (17) comprises means for moving the treatment light source (18).

5. A skin treatment system as claimed in claim 1, wherein the light based skin treatment device (10, 20, 30) comprises optical elements (11, 12, 14) for focusing the treatment light beam (21) outside the device (10, 20, 30) and inside the hair or skin tissue and wherein the exit location control unit (17) comprises means for configuring a position and/or orientation of at least one of the optical elements (11, 12, 14).

6. A skin treatment system as claimed in claim 1, wherein the exit location control unit (17) is arranged for scanning the treatment light beam over an area of skin (35), and for deactivating the treatment light source (18) when the exit location control unit (17) is arranged such that activation of the treatment light source (18) would have caused the treatment light beam (21) to pass through the damaged areas.

7. A skin treatment system as claimed in claim 1, wherein the control circuit (16) is arranged to analyze at least two detection signals from the detection unit (15), generated with the detection light beam (21) passing through the exit window (14) at the same or almost the same position and at at least two respective moments in time, and to declare a certain area at the outer surface as damaged if the at least two detection signals represent a similar aberrant scattering profile of the detection light beam (21) at the outer surface.

8. A skin treatment system as claimed in claim 1, wherein the detection light source (18), the detection unit (15) and the control circuit (16) are comprised in the light based skin treatment device (10, 20), the detection unit (15) being arranged for detecting the detection light beam (21) after having interacted with the hair or skin tissue.

9. A skin treatment system as claimed in claim 1, further comprising a docking station (40) for receiving the light based skin treatment device (30), the docking station (40) comprising the detection unit (15), the docking station (40) being operative to provide a coupling between the detection unit (15) and the control unit (16), at least when the light based skin treatment device (30) is received by the docking station (40).

10. A skin treatment system as claimed in claim 9, wherein the docking station (40) further comprises the detection light source (18).

11. A skin treatment system as claimed in claim 9, wherein the docking station (40) further comprises a cleaning unit for cleaning the outer surface of the exit window (14).

12. A skin treatment system as claimed in claim 1, wherein the treatment light beam (21) is provided for treating the skin (35) by laser induced optical breakdown (LIOB) of hair or skin tissue.

13. A skin treatment system as claimed in claim 12, wherein the treatment light beam (21) is provided for LIOB caused cutting of hair (31).

14. A skin treatment system as claimed in claim 12, wherein the treatment light beam (21) is provided for LIOB caused skin rejuventation.

15. A docking station (40) for use in a system as claimed in claim 1 and being arranged for receiving the light based skin treatment device (30), the docking station (40) comprising a light detection unit (15) and being operative to provide a coupling between the detection unit (15) and the control circuit (16), at least when the light based skin treatment device (30) is received by the docking station (40).

## Patentansprüche

1. Hautbehandlungssystem, umfassend:
- eine lichtbasierte Hautbehandlungseinrichtung (10, 20, 30) mit:
einer Behandlungslichtquelle (18), um einen Behandlungslichtstrahl (21) zur Behandlung von Haar- oder Hautgewebe vorzusehen,
einem transparenten Austrittsfenster (14), damit der Behandlungslichtstrahl (21) aus der Einrichtung (10, 20, 30) austreten kann, sowie
einer Austrittspositionssteuereinheit (17) zur Steuerung einer Position, an welcher der Behandlungslichtstrahl (21) durch das Austrittsfenster (14) hindurchtritt,
- eine Detektionslichtquelle (18) zum Vorsehen eines Detektionslichtstrahls (21),
- eine Detektionseinheit (15) zum Detektieren des Detektionslichtstrahls (21), nachdem dieser durch das Austrittsfenster (14) mindestens einmal zumindest teilweise hindurchgetreten ist,
**gekennzeichnet durch**
- einen Steuerkreis (16), der so eingerichtet ist, dass er ein Detektionssignal von der Detektionseinheit (15) analysiert, um beschädigte Bereiche an einer Außenfläche des Austrittsfensters (14) festzustellen, und die Austrittspositionssteuereinheit (17) steuert, um zu verhindern, dass der Behandlungslichtstrahl (18) **durch** die beschädigten Bereiche hindurchgeht.

2. Hautbehandlungssystem nach Anspruch 1, wobei es sich bei der Behandlungslichtquelle und der Detektionslichtquelle um die gleiche Lichtquelle (18) handelt.

3. Hautbehandlungssystem nach Anspruch 2, wobei es sich bei dem Behandlungslichtstrahl und dem Detektionslichtstrahl um den gleichen einfallenden Lichtstrahl (21) handelt.

4. Hautbehandlungssystem nach Anspruch 1, wobei die Austrittspositionssteuereinheit (17) Mittel umfasst, um die Behandlungslichtquelle (18) zu bewegen.

5. Hautbehandlungssystem nach Anspruch 1, wobei die lichtbasierte Hautbehandlungseinrichtung (10, 20, 30) optische Elemente (11, 12, 14) umfasst, um den Behandlungslichtstrahl (21) außerhalb der Einrichtung (10, 20, 30) und innerhalb des Haar- oder Hautgewebes zu fokussieren, und wobei die Austrittspositionssteuereinheit (17) Mittel umfasst, um eine Position und/oder Ausrichtung von mindestens einem der optischen Elemente (11, 12, 14) zu konfigurieren.

6. Hautbehandlungssystem nach Anspruch 1, wobei die Austrittspositionssteuereinheit (17) so eingerichtet ist, dass sie den Behandlungslichtstrahl über einen Hautbereich (35) scannt und die Behandlungslichtquelle (18) deaktiviert, wenn die Austrittspositionssteuereinheit (17) so eingerichtet ist, dass eine Aktivierung der Behandlungslichtquelle (18) bewirkt haben würde, dass der Behandlungslichtstrahl (21) durch die beschädigten Bereiche hindurchgeht.

7. Hautbehandlungssystem nach Anspruch 1, wobei der Steuerkreis (16) so eingerichtet ist, dass er mindestens zwei Detektionssignale von der Detektionseinheit (15) analysiert, die mit dem Detektionslichtstrahl (21) erzeugt werden, der durch das Austrittsfenster (14) an der gleichen oder nahezu der gleichen Position und zu mindestens zwei jeweiligen Zeitpunkten hindurchgeht, und einen bestimmten Bereich an der Außenfläche als beschädigt erklärt, wenn die mindestens zwei Detektionssignale ein ähnliches fehlerbehaftetes Streuprofil des Detektionslichtstrahls (21) an der Außenfläche darstellen.

8. Hautbehandlungssystem nach Anspruch 1, wobei die Detektionslichtquelle (18), die Detektionseinheit (15) und der Steuerkreis (16) in der lichtbasierten Hautbehandlungseinrichtung (10, 20) enthalten sind, wobei die Detektionseinheit (15) so eingerichtet ist, dass sie den Detektionslichtstrahl (21) nach Interagieren mit dem Haar- oder Hautgewebe detektiert.

9. Hautbehandlungssystem nach Anspruch 1, das weiterhin eine Docking-Station (40) zur Aufnahme der lichtbasierten Hautbehandlungseinrichtung (30) umfasst, wobei die Docking-Station (40) die Detektionseinheit (15) umfasst, wobei die Docking-Station (40) so arbeitet, dass sie, zumindest wenn die lichtbasierte Hautbehandlungseinrichtung (30) von der Docking-Station (40) aufgenommen wird, eine Kopplung zwischen der Detektionseinheit (15) und dem Steuerkreis (16) vorsieht.

10. Hautbehandlungssystem nach Anspruch 9, wobei die Docking-Station (40) weiterhin die Detektionslichtquelle (18) umfasst.

11. Hautbehandlungssystem nach Anspruch 9, wobei die Docking-Station (40) weiterhin eine Reinigungseinheit zur Reinigung der Außenfläche des Austrittsfensters (14) umfasst.

12. Hautbehandlungssystem nach Anspruch 1, wobei der Behandlungslichtstrahl (21) zur Behandlung der Haut (35) durch Laser-induzierten optischen Durchbruch (LIOB) von Haar- oder Hautgewebe vorgesehen wird.

13. Hautbehandlungssystem nach Anspruch 12, wobei der Behandlungslichtstrahl (21) zum LIOB-bewirkten Schneiden von Haar (31) vorgesehen ist.

14. Hautbehandlungssystem nach Anspruch 12, wobei der Behandlungslichtstrahl (21) zur LIOB-bewirkten Hautverjüngung vorgesehen ist.

15. Docking-Station (40) zur Verwendung in einem System nach Anspruch 1, die so angeordnet ist, dass sie die lichtbasierte Hautbehandlungseinrichtung (30) aufnimmt, wobei die Docking-Station (40) eine Lichtdetektionseinheit (15) umfasst und so arbeitet, dass sie, zumindest wenn die lichtbasierte Hautbehandlungseinrichtung (30) von der Docking-Station (40) aufgenommen wird, eine Kopplung zwischen der Detektionseinheit (15) und dem Steuerkreis (16) vorsieht.

## Revendications

1. Système de traitement de peau, comprenant :
- un dispositif de traitement de peau à base de lumière (10, 20, 30) comprenant
une source de lumière de traitement (18) pour fournir un faisceau de lumière de traitement (21) pour traiter un tissu pileux ou cutané,
une fenêtre de sortie transparente (14) pour permettre au faisceau de lumière de traitement (21) de quitter le dispositif (10, 20, 30), et
une unité de commande de localisation de sortie (17) pour commander une position pour le faisceau de lumière de traitement (21) pour passer à travers la fenêtre de sortie (14),
une source de lumière de détection (18) pour fournir un faisceau de lumière de détection (21),
- une unité de détection (15) pour détecter le faisceau de lumière de détection (21) après être passé au moins partiellement à travers la fenêtre de sortie (14) au moins une fois, **caractérisé par**
- un circuit de commande (16) agencé pour analyser un signal de détection à partir de l'unité de détection (15) pour trouver des zones endommagées sur une surface extérieure de la fenêtre de sortie (14), et pour commander l'unité de commande de localisation de sortie (17) afin d'éviter que le faisceau de lumière de traitement (18) passe à travers les zones endommagées.

2. Système de traitement de peau selon la revendication 1, dans lequel la source de lumière de traitement et la source de lumière de détection sont la même source de lumière (18).

3. Système de traitement de peau selon la revendication 2, dans lequel le faisceau de lumière de traitement et le faisceau de lumière de détection sont le même faisceau de lumière incident (21).

4. Système de traitement de peau selon la revendication 1, dans lequel l'unité de commande de localisation de sortie (17) comprend des moyens pour déplacer la source de lumière de traitement (18).

5. Système de traitement de peau selon la revendication 1, dans lequel le dispositif de traitement de peau à base de lumière (10, 20, 30) comprend des éléments optiques (11, 12, 14) pour concentrer le faisceau de lumière de traitement (21) à l'extérieur du dispositif (10, 20, 30) et à l'intérieur du tissu pileux ou cutané et dans lequel l'unité de commande de localisation de sortie (17) comprend des moyens pour configurer une position et/ou une orientation d'au moins un des éléments optiques (11, 12, 14).

6. Système de traitement de peau selon la revendication 1, dans lequel l'unité de commande de localisation de sortie (17) est agencée pour faire balayer le faisceau de lumière de traitement sur une zone de peau (35), et pour désactiver la source de lumière de traitement (18) lorsque l'unité de commande de localisation de sortie (17) est agencée de sorte que l'activation de la source de lumière de traitement (18) entraîne le passage du faisceau de lumière de traitement (21) à travers les zones endommagées.

7. Système de traitement de peau selon la revendication 1, dans lequel le circuit de commande (16) est agencé pour analyser au moins deux signaux de détection à partir de l'unité de détection (15), générés avec le faisceau de lumière de détection (21) passant à travers la fenêtre de sortie (14) à la même ou presque la même position et à au moins deux moments respectifs, et pour déclarer qu'une certaine zone sur la surface extérieure est endommagée si les au moins deux signaux de détection représentent un profil similaire de diffusion aberrante du faisceau de lumière de détection (21) sur la surface extérieure.

8. Système de traitement de peau selon la revendication 1, dans lequel la source de lumière de détection (18), l'unité de détection (15) et le circuit de commande (16) sont compris dans le dispositif de traitement de peau à base de lumière (10, 20), l'unité de détection (15) étant agencée pour détecter le faisceau de lumière de détection (21) après avoir interagi avec le tissu pileux ou cutané.

9. Système de traitement de peau selon la revendication 1, comprenant en outre un socle d'accueil (40) pour recevoir le dispositif de traitement de peau à base de lumière (30), le socle d'accueil (40) comprenant l'unité de détection (15), le socle d'accueil (40) étant fonctionnel pour fournir un accouplement entre l'unité de détection (15) et l'unité de commande (16), au moins lorsque le dispositif de traitement de peau à base de lumière (30) est reçu par le socle d'accueil (40).

10. Système de traitement de peau selon la revendication 9, dans lequel le socle d'accueil (40) comprend en outre la source de lumière de détection (18).

11. Système de traitement de peau selon la revendication 9, dans lequel le socle d'accueil (40) comprend en outre une unité de nettoyage pour nettoyer la surface extérieure de la fenêtre de sortie (14).

12. Système de traitement de peau selon la revendication 1, dans lequel le faisceau de lumière de traitement (21) est fourni pour traiter la peau (35) par décomposition optique induite par laser (« Laser Induced Optical Breakdown » ou LIOB) de tissu pileux ou cutané.

13. Système de traitement de peau selon la revendication 12, dans lequel le faisceau de lumière de traitement (21) est fourni pour la coupe entraînée par LIOB de poil (31).

14. Système de traitement de peau selon la revendication 12, dans lequel le faisceau de lumière de traitement (21) est fourni pour le rajeunissement de peau entraîné par LIOB.

15. Socle d'accueil (40) pour l'utilisation dans un système selon la revendication 1 et étant agencé pour recevoir le dispositif de traitement de peau à base de lumière (30), le socle d'accueil (40) comprenant une unité de détection de lumière (15) et étant fonctionnel pour fournir un accouplement entre l'unité de détection (15) et le circuit de commande (16), au moins lorsque le dispositif de traitement de peau à base de lumière (30) est reçu par le socle d'accueil (40).
